# EUROPEAN PATENT SPECIFICATION

(11) **EP 3 212 155 B1**
(45) Date of publication and mention of the grant of the patent: **03.04.2019**
(21) Application number: 15784065.3
(22) Date of filing: 22.10.2015
(51) Int. Cl.: A61K 8/34, A61K 8/35, A61K 8/37, A61Q 15/00

(54) **METHOD OF CONTROLLING SWEAT PRODUCTION**
VERFAHREN ZUR KONTROLLE DER SCHWEISSPRODUKTION
PROCÉDÉ DE RÉGULATION DE LA PRODUCTION DE SUEUR

(30) Priority: 30.10.2014 EP 14191106
(43) Date of publication of application: 06.09.2017
(73) Proprietor: Unilever PLC, London, Greater London EC4Y 0DY (GB); Unilever N.V., 3013 AL Rotterdam (NL)
(72) Inventor: BOVELL, Douglas, Bishopton PA7 5BL (GB); EVANS, Richard, Livesey, Wirral Merseyside CH63 3JW (GB); HARDING, Clive, Roderick, Wirral Merseyside CH63 3JW (GB); HARICHIAN, Bijan, Trumbull CT Connecticut 06611 (US); ROBERTSON, Jennifer, Falkirk Stirlingshire FK1 5RF (GB); STOTT, Ian, Peter, Wirral Merseyside CH63 3JW (GB)
(74) Representative: Whaley, Christopher
(86) International application number: PCT/EP2015/074488
(87) International publication number: WO 2016/066518

(56) References cited:
- EP-A2- 2 100 591
- FR-A1- 2 758 087
- FR-A1- 2 934 778

## Description

The present invention is in the field of antiperspirancy, in particular methods of achieving antiperspirancy without gland blockage. The invention is of particular value in treating heavy sweating and/or hyperhydrosis.

There have been several publications published relating to the reduction of sweating using actives that do not block the sweat glands or pores.

WO 02/011690 (Unilever) discloses an antiperspirancy method whereby calcium channels with the secretory coil cells of the eccrine glands are blocked, thereby controlling sweat production at its source.

US 8,618,160 B2 (Rose U) discloses wipes containing glycopyrrolate, a muscarinic anticholinergic, as a treatment for hyperhidrosis. The document FR2934778 is also relevant in this context.

Certain anticholinergic drugs have also been used to treat hyperhidrosis, with varying degrees of success. These drugs include Ditropan® (oxybutynin), Probanthine® (propantheline bromide) and Cogentin® (benzotropine).

US 2008/0207737 (Zinger) discloses a topical composition for antiperspirant benefit comprising varying levels of oxybutynin.

In a first aspect of the invention, there is provided a cosmetic method of reducing perspiration comprising the topical application of a composition comprising a non-pore blocking antiperspirant agent of general formula I: Ar-CHₐCHₐ-X, wherein Ar is an aryl group, a is 1 or 2, and X is a sub-structure of formula CH(OH)-Y or CO.Z, wherein Y is a C₁-C₁₀ hydroxyalkyl group and Z is a C₁-C₁₀ hydroxyalkyl group or a C₁-C₁₀ oxyalkyl group.

In a second aspect of the invention, there is provided a non-pore blocking antiperspirant agent as defined in the first aspect of the invention for non-therapeutic use as a treatment for perspiration, in particular excessive perspiration.

In a third aspect of the invention, there is provided an antiperspirant composition comprising a non-pore blocking antiperspirant agent selected from 6-gingerol, 6-gingerdiol or C₁-C₄ alkyl cinnamate and a carrier fluid comprising a C₂-C₆ vicinal diol.

In a fourth aspect of the invention, there is provided an antiperspirant composition as described in the third aspect of the invention and a dispenser for said composition, the dispenser comprising containment means and application means for the composition.

In the cosmetic method described in the first aspect of the invention, the composition preferably comprises a carrier fluid and more preferably a carrier fluid comprising a C₂-C₆ vicinal diol.

The aforementioned cosmetic method is a non-therapeutic method. In non-therapeutic cosmetic methods, the non-pore blocking antiperspirant agent is typically applied as part of a cosmetic composition comprising a cosmetically acceptable carrier fluid.

In the second aspect of the invention, the non-pore blocking antiperspirant agent as defined in the first aspect of the invention may be used for the cosmetic or therapeutic treatment of perspiration, in particular excessive perspiration. Preferably, the non-pore blocking antiperspirant agent is used in conjunction with a carrier fluid and more preferably with a carrier fluid comprising a C₂-C₆ vicinal diol.

In the third aspect of the invention, preferred compositions comprise a non-pore blocking antiperspirant agent selected from 6-gingerol or 6-gingerdiol and a carrier fluid comprising a C₂-C₆ vicinal diol.

Herein, excessive perspiration should be understood to refer to that condition otherwise known as hyperhidrosis.

Herein, all percentages, parts, and ratios are by weight, unless otherwise indicated.

Herein, references to an amount of a compound or an active refer to the total amount of compounds or actives of the type indicated.

Herein, "application" and "applied" relate to application to the surface of the human body, in particular the underarm regions, unless the context dictates otherwise.

The invention typically involves the topical application of the non-pore blocking antiperspirant agent to the underarm regions of the human body, otherwise known as the axillae. From there, it is believed that the antiperspirant agent permeates to the secretory coil of the sweat glands, said permeation generally being enhanced by other components with which the antiperspirant agent is formulated. Without wishing to be bound by theory, it is believed that the antiperspirant agent reduces sweat production at its source, the sweat glands themselves.

The non-pore blocking antiperspirant agent used in accordance with the present invention is of general formula I (Ar-CHₐCHₐ-X) as previously defined.

Herein, an "aryl" group is an optionally substituted phenyl group. Preferably, *para* to the X group and at solely one position *meta* to the X group, the aryl group is non-substituted or has a substituent selected from OH or OCH₃ and at all other positions the aryl group is non-substituted.

For the avoidance of doubt, a non-substituted position on an aryl group in Formula I merely bears an H atom.

For the avoidance of doubt, when "a" in Formula I equals 1, a double bond exists between the indicated carbon atoms and when "b" in Formula I equals 2, a single bond exists between the indicated carbon atoms.

When X in general formula I is a sub-structure of formula CH(OH)-Y, Y is preferably of formula CH₂CH(OH)R, wherein R is a C₁-C₆ alkyl group (preferably a C₅ linear, unbranched alkyl group).

When X in general formula I is a sub-structure of formula CO.Z, Z is preferably of formula CH₂CH(OH)R, wherein R is a C₁-C₆ alkyl group (preferably a C₅ linear, unbranched alkyl group) or Z is a C₁-C₄ oxyalkyl group.

Herein, an "oxyalkyl group" should be understood to be an O atom followed by a non-substituted alkyl group, typically linear and unbranched.

In preferred embodiments, the non-pore blocking antiperspirant agent is selected from 6-gingerol, 6-gingerdiol, or a C₁-C₄ alkyl cinnamate (preferably ethyl cinnamate).

In particularly preferred embodiments, the non-pore blocking antiperspirant agent is selected from 6-gingerol or 6-gingerdiol.

6-gingerol has the systematic name (5*S*)-5-hydroxy-1-(4-hydroxy-3-methoxyphenyl)-3-decanone.

6-gingerdiol has the systematic name (5S)-1-(4-hydroxy-3-methoxyphenyl)-decane-3,5-diol.

The non-pore blocking antiperspirant agent is preferably used from a composition comprising it at a level of from 0.1 to 20%, preferably from 0.2 to 10% and more preferably from 0.5 to 5%.

Compositions used in accordance with the present invention may optionally include pore-blocking antiperspirant agents, including conventional astringent aluminium-containing antiperspirant salts.

Such conventional astringent aluminium-containing antiperspirant salts include aluminium, zirconium and mixed aluminium/ zirconium salts, including both inorganic salts, salts with organic anions and complexes. Preferred astringent salts include aluminium, zirconium and aluminium/zirconium halides and halohydrate salts, such as chlorohydrates. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in US 3,792,068 (Procter and Gamble Co.). Zinc phenol sulphonate may also be used, preferably at up to 3% by weight of the composition.

When employed, preferred levels of incorporation of conventional astringent aluminium-containing antiperspirant salts are from 0.5% to 60%, particularly from 5% to 40%, and especially from 5% or 10% to 30% or 35% of the composition.

In certain preferred embodiments of the invention, conventional astringent aluminium-containing antiperspirant salts are not employed.

The carrier fluid used in conjunction with the present invention should be cosmetically acceptable and aid in the delivery of the non-pore blocking antiperspirant agent to the surface of the human body. The non-pore blocking antiperspirant agent is typically suspended or dissolved in the carrier fluid.

The carrier fluid may comprise from 10 to 99% of the composition.

In preferred aspects of the present invention, the carrier fluid for the non-pore blocking antiperspirant agent comprises a C₂-C₆ vicinal diol.

Herein, the C₂-C₆ vicinal diol is a compound with from 2 to 6 carbon atoms in which two neighbouring carbon atoms bear hydroxyl groups.

Examples of C₂-C₆ vicinal diols include ethylene glycol, propylene glycol, glycerol and hexane-1,2-diol.

Preferably, the C₂-C₆ vicinal diol excludes compounds comprising more than 3 hydroxyl groups.

Preferably the C₂-C₆ vicinal diol is a C₂-C₄ vicinal diol, i.e. a compound with from 2 to 4 carbon atoms in which two neighbouring carbon atoms bear hydroxyl groups.

Preferably, the C₂-C₆ or C₂-C₄ vicinal diol excludes compounds comprising more than 3 hydroxyl groups.

Preferably, the C₂-C₆ vicinal diol excludes mono-saccharides and reduced mono-saccharides.

The content of C₂-C₆ vicinal diol in compositions suitable for use in accordance with the invention is preferably from 1 to 50%, more preferably from 2 to 40% and most preferably from 5 to 25%.

A preferred additional component of the carrier fluid is ethanol. This may comprise up to 80% of the total composition, but is typically restricted to less than 70%, and often less than 60%. When employed ethanol typically comprises at least 10%, preferably at least 20%, and more preferably at least 40% by weight of total composition. Each of these preferred minimum levels of ethanol may be limited by the aforementioned maximum levels of ethanol.

A preferred additional component of the carrier fluid is water, especially when employed in conjunction with ethanol, to give an aqueous ethanol carrier fluid. Water may comprise up to 90% of the total composition, but is typically restricted to less than 60%, and often less than 50%. When employed water typically comprises at least 10%, preferably at least 20%, and more preferably at least 30% by weight of total composition. Each of these preferred minimum levels of water may be limited by the aforementioned maximum levels of water.

A preferred additional component is a fragrance, typically at a level of from 0.1 to 5% of the total composition. In compositions also comprising water, the fragrance is preferably accompanied by a fragrance solubiliser, typically a non-ionic surfactant used a concentration of from 0.1 to 5% of the total composition.

A further optional component is an organic anti-microbial agent. Such agents may be selected from any of those known in the art, provided reasonable skill is used to avoid any incompatibilities. An organic anti-microbial agent is a particularly preferred additional component when the composition used does not include a conventional, astringent aluminium containing antiperspirant salt. When employed, organic anti-microbial agents are typically used at a level of from 0.1 to 5% by weight of the composition.

Thickening agents may be employed in compositions of the invention. Such agents increase the viscosity of or solidify the carrier fluid in which the antiperspirant agent is typically suspended or dissolved.

Thickening agents may be selected from any of those known in the art, provided reasonable skill is used to avoid any incompatibilities. A preferred class of thickeners, especially for compositions also comprising water, are hydroxyalkyl celluloses, such as hydroxypropyl cellulose. When employed, thickening agents are typically used at a level of from 0.1 to 40%. When a hydroxyalkyl cellulose thickening agent is employed, this is typically used at a level of from 0.2 to 10% by weight.

Dispensers suitable for use with the present invention comprise containment means and application means for the composition. They also typically comprise means for getting the composition from its containment means to its application means.

The containment means is preferably a reservoir for the composition and the application means is preferably a surface from which the composition may be massaged into the skin.

Preferred dispensers are able to function without the consumer needing to touch the composition with his or her hands in order for it to be applied, this feature enhancing dosing accuracy.

### Examples

The compositions detailed in Table 1 may be prepared by methods known in the art. The pH of the resulting composition is adjusted to 6 to 7 with 0.2N sodium hydroxide solution. The level of hydroxypropyl cellulose may be adjusted to gain the desired level of viscosity for the composition.

**Table 1**

| | **Example** | | | | |
|---|---|---|---|---|---|
| | **1** | **2** | **3** | **4** | **5** |
| 6-gingerol | 1.0 | 0.5 | -- | -- | -- |
| 6-gingerdiol | -- | -- | 1.0 | 0.5 | -- |
| Ethyl cinnamate | -- | -- | -- | -- | 1.0 |
| Hydroxypropyl cellulose | 1.7 | 1.7 | 1.5 | 1.0 | 1.0 |
| Propylene glycol | 10.0 | 10.0 | 12.0 | 8.0 | 8.0 |
| Glycerol | 1.0 | 1.0 | 0.8 | 1.2 | 1.2 |
| Ethanol | 50 | 50 | 60 | 40 | 40 |
| Water | To 100 | To 100 | To 100 | To 100 | To 100 |

## Claims

1. The non-therapeutic use of a non-pore blocking antiperspirant agent of general formula I for reducing perspiration, general formula I being:
Ar-CHₐCHₐ-X
wherein Ar is an aryl group, a is 1 or 2, and X is a sub-structure of formula CH(OH)-Y or CO.Z, wherein Y is a C₁-C₁₀ hydroxyalkyl group and Z is a C₁-C₁₀ hydroxyalkyl group or a C₁-C₁₀ oxyalkyl group.

2. The non therapeutic use according to claim 1, wherein the non-pore blocking antiperspirant agent of general formula I has an aryl group such that *para* to the X group and at solely one position *meta* to the X group, the aryl group is non-substituted or has a substituent selected from OH or OCH₃ and at all other positions the aryl group is non-substituted.

3. The non therapeutic use according to claim 1 or claim 2, wherein X in general formula I is as a sub-structure of formula CH(OH)-Y' or CO.Z, wherein Y' is of formula CH₂CH(OH)R, wherein R is a C₁-C₆ alkyl group (preferably a C₅ linear, unbranched alkyl group), and Z is as defined in claim 1.

4. A cosmetic method of reducing perspiration comprising the topical application of a composition comprising a non-pore blocking antiperspirant agent of general formula I:
Ar-CHₐCHₐ-X
wherein Ar is an aryl group, a is 1 or 2, and X is a sub-structure of formula CH(OH)-Y or CO.Z', wherein Y is a C₁-C₁₀ hydroxyalkyl group and Z' is of formula CH₂CH(OH)R, wherein R is a C₁-C₆ alkyl group (preferably a C₅ linear, unbranched alkyl group) or is a C₁-C₄ oxyalkyl group.

5. A cosmetic method according to claim 4, wherein the non-pore blocking antiperspirant agent is selected from 6-gingerol, 6-gingerdiol, or ethyl cinnamate.

6. A cosmetic method according to claim 5, wherein the non-pore blocking antiperspirant agent is selected from 6-gingerol or 6-gingerdiol.

7. A cosmetic method according to claim 4 or claim 5, wherein a conventional astringent aluminium-containing antiperspirant salt is not also applied.

8. A non-pore blocking antiperspirant agent as described in any of the preceding claims for use as a treatment for excessive perspiration.

9. An antiperspirant composition comprising a non-pore blocking antiperspirant agent selected from 6-gingerol, 6-gingerdiol or C₁-C₄ alkyl cinnamate and a carrier fluid comprising a C₂-C₆ vicinal diol.

10. A composition according to claim 9, wherein the non-pore blocking antiperspirant agent comprises from 0.2 to 10% of the composition, the carrier fluid comprises from 10 to 99% of the composition, and the C₂-C₆ vicinal diol from 2 to 40% of the composition, all percentages being by weight.

11. A composition according to claim 9 or claim 10, comprising a C₂-C₄ vicinal diol.

12. A composition according to any of claims 9 to 11, wherein the carrier fluid comprises water and ethanol.

13. A composition according to any of claims 9 to 12, wherein the non-pore blocking antiperspirant agent selected from 6-gingerol or 6-gingerdiol.

14. A product comprising a composition according to any of claims 9 to 13 and dispenser for said composition, the dispenser comprising containment means and application means for the composition.

15. A product according to claim 14, wherein the dispenser is able to function without the consumer needing to touch the composition with his or her hands in order for it to be applied.

## Patentansprüche

1. Nicht-therapeutische Verwendung eines keine Poren blockierenden Antitranspirantmittels der allgemeinen Formel I zur Verringerung der Transpiration, wobei die allgemeine Formel I ist:
Ar-CHₐCHₐ-X,
worin Ar eine Arylgruppe ist, a 1 oder 2 ist und X eine Substruktur der Formel CH(OH)-Y oder CO.Z ist, wobei Y eine C₁-C₁₀-Hydroxyalkylgruppe ist und Z eine C₁-C₁₀-Hydroxyalkylgruppe oder eine C₁-C₁₀-Oxyalkylgruppe ist.

2. Nicht-therapeutische Verwendung nach Anspruch 1, wobei das keine Poren blockierende Antitranspirantmittel der allgemeinen Formel I eine Arylgruppe derartig aufweist, dass die Arylgruppe, para zu der X-Gruppe und lediglich in einer Position meta zu der X-Gruppe, nicht-substituiert ist oder einen Substituenten aufweist, der aus OH oder OCH₃ ausgewählt ist, und die Arylgruppe in allen anderen Positionen nicht-substituiert ist.

3. Nicht-therapeutische Verwendung nach Anspruch 1 oder Anspruch 2, wobei X in der allgemeinen Formel I als eine Substruktur der Formel CH(OH)-Y' oder CO.Z vorliegt, wobei Y' die Formel CH₂CH(OH)R aufweist, wobei R eine C₁-C₆-Alkylgruppe (vorzugsweise eine lineare unverzweigte C₅-Alkylgruppe) ist und Z die Definition des Anspruchs 1 aufweist.

4. Kosmetisches Verfahren zur Verringerung der Transpiration, umfassend das topische Auftragen einer Zusammensetzung, umfassend ein nicht Poren blockierendes Antitranspirantmittel der allgemeinen Formel I:
Ar-CHₐCHₐ-X,
worin Ar eine Arylgruppe ist, a 1 oder 2 ist und X eine Substruktur der Formel CH(OH)-Y oder CO.Z' ist, wobei Y eine C₁-C₁₀-Hydroxyalkylgruppe ist und Z' die Formel CH₂CH(OH)R aufweist, wobei R eine C₁-C₆-Alkylgruppe (vorzugsweise eine lineare, unverzweigte C₅-Alkylgruppe) oder eine C₁-C₄-Oxyalkylgruppe ist.

5. Kosmetisches Verfahren nach Anspruch 4, wobei das keine Poren blockierende Antitranspirantmittel aus 6-Gingerol, 6-Gingerdiol oder Ethylcinnamat ausgewählt ist.

6. Kosmetisches Verfahren nach Anspruch 5, wobei das keine Poren blockierende Antitranspirantmittel aus 6-Gingerol oder 6-Gingerdiol ausgewählt ist.

7. Kosmetisches Verfahren nach Anspruch 4 oder Anspruch 5, wobei ein konventionelles astringierendes Aluminium enthaltendes Antitranspirantsalz ebenfalls nicht aufgetragen wird.

8. Keine Poren blockierendes Antitranspirantmittel, wie in irgendeinem der vorhergehenden Ansprüche beschrieben, zur Verwendung bei der Behandlung exzessiver Transpiration.

9. Antitranspirantzusammensetzung, umfassend ein keine Poren blockierendes Antitranspirantmittel, ausgewählt aus 6-Gingerol, 6-Gingerdiol oder C₁-C₄-Alkylcinnamat und einer Trägerflüssigkeit, umfassend ein vicinales C₂-C₆-Diol.

10. Zusammensetzung nach Anspruch 9, wobei das keine Poren blockierende Antitranspirantmittel von 0,2 bis 10% der Zusammensetzung, die Trägerflüssigkeit von 10 bis 99% der Zusammensetzung und das vicinale C₂-C₆-Diol von 2 bis 40% der Zusammensetzung umfasst, wobei sich alle Prozentangaben auf das Gewicht beziehen.

11. Zusammensetzung nach Anspruch 9 oder Anspruch 10, umfassend ein vicinales C₂-C₄-Diol.

12. Zusammensetzung nach irgendeinem der Ansprüche 9 bis 11, wobei die Trägerflüssigkeit Wasser und Ethanol umfasst.

13. Zusammensetzung nach irgendeinem der Ansprüche 9 bis 12, wobei das keine Poren blockierende Antitranspirantmittel aus 6-Gingerol oder 6-Gingerdiol ausgewählt ist.

14. Produkt, umfassend eine Zusammensetzung nach irgendeinem der Ansprüche 9 bis 13 und einen Spender für diese Zusammensetzung, wobei der Spender ein Aufnahmemittel und ein Auftragsmittel für die Zusammensetzung umfasst.

15. Produkt nach Anspruch 14, wobei der Spender funktionsfähig ist, ohne dass der (die) Verbraucher(in) mit seinen oder ihren Händen die Zusammensetzung berühren muss, um sie aufzutragen.

## Revendications

1. Utilisation non-thérapeutique d'un agent antiperspirant ne bloquant pas les pores de formule générale I pour réduire la transpiration, la formule générale I étant :
Ar-CHₐCHₐ-X
où Ar est un groupe aryle, a est égal à 1 ou 2, et X est une sous-structure de formule CH(OH)-Y ou CO.Z, où Y est un groupe hydroxyalkyle en C₁-C₁₀ et Z est un groupe hydroxyalkyle en C₁-C₁₀ ou un groupe oxyalkyle en C₁-C₁₀.

2. Utilisation non-thérapeutique selon la revendication 1, dans laquelle l'agent antiperspirant ne bloquant pas les pores de formule générale I présente un groupe aryle tel que *para* par rapport au groupe X et à seulement une position *méta* par rapport au groupe X, le groupe aryle n'est pas substitué ou présente un substituant choisi parmi OH ou OCH₃ et à toutes les autres positions le groupe aryle n'est pas substitué.

3. Utilisation non-thérapeutique selon la revendication 1 ou la revendication 2, dans laquelle X dans la formule générale I est comme une sous-structure de formule CH(OH)-Y' ou CO.Z, où Y' est de la formule CH₂CH(OH)R, où R est un groupe alkyle en C₁-C₆ (de préférence un groupe alkyle linéaire, non-ramifié en C₅), et Z est comme défini dans la revendication 1.

4. Procédé cosmétique de réduction de la transpiration comprenant l'application topique d'une composition comprenant un agent antiperspirant ne bloquant pas les pores de formule générale I :
Ar-CHₐCHₐ-X
où Ar est un groupe aryle, a est égal à 1 ou 2, et X est une sous-structure de formule CH(OH)-Y ou CO.Z', où Y est un groupe hydroxyalkyle en C₁-C₁₀ et Z' est de la formule CH₂CH(OH)R, où R est un groupe alkyle en C₁-C₆ (de préférence un groupe alkyle linéaire, non-ramifié en C₅) ou est un groupe oxyalkyle en C₁-C₄.

5. Procédé cosmétique selon la revendication 4, dans lequel l'agent antiperspirant ne bloquant pas les pores est choisi parmi le 6-gingérol, 6-gingerdiol, ou cinnamate d'éthyle.

6. Procédé cosmétique selon la revendication 5, dans lequel l'agent antiperspirant ne bloquant pas les pores est choisi parmi le 6-gingérol ou 6-gingerdiol.

7. Procédé cosmétique selon la revendication 4 ou la revendication 5, dans lequel un sel antiperspirant contenant de l'aluminium astringent classique n'est également pas appliqué.

8. Agent antiperspirant ne bloquant pas les pores comme décrit dans l'une quelconque des revendications précédentes pour une utilisation comme un traitement pour transpiration excessive.

9. Composition d'antiperspirant comprenant un agent antiperspirant ne bloquant pas les pores choisi parmi le 6-gingérol, 6-gingerdiol ou cinnamate d'alkyle en C₁-C₄ et un fluide porteur comprenant un diol vicinal en C₂-C₆.

10. Composition selon la revendication 9, dans laquelle l'agent antiperspirant ne bloquant pas les pores comprend de 0,2 à 10 % de la composition, le fluide porteur comprend de 10 à 99 % de la composition, et le diol vicinal en C₂-C₆ de 2 à 40 % de la composition, tous les pourcentages étant en masse.

11. Composition selon la revendication 9 ou la revendication 10, comprenant un diol vicinal en C₂-C₄.

12. Composition selon l'une quelconque des revendications 9 à 11, dans laquelle le fluide porteur comprend de l'eau et de l'éthanol.

13. Composition selon l'une quelconque des revendications 9 à 12, dans laquelle l'agent antiperspirant ne bloquant pas les pores est choisi parmi le 6-gingérol ou 6-gingerdiol.

14. Produit comprenant une composition selon l'une quelconque des revendications 9 à 13 et distributeur pour ladite composition, le distributeur comprenant un moyen de confinement et un moyen d'application pour la composition.

15. Produit selon la revendication 14, dans lequel le distributeur peut fonctionner sans que l'utilisateur ait besoin de toucher la composition avec ses mains pour qu'elle soit appliquée.
